# EUROPÄISCHE PATENTANMELDUNG

(11) **EP 1 780 287 A1**
(43) Veröffentlichungstag der Anmeldung: **02.05.2007**
(21) Anmeldenummer: 05023420.2
(22) Anmeldetag: 26.10.2005
(51) Int. Cl.: C12Q 1/34, C12Q 1/48, G01N 33/68

(54) **In vitro Verfahren zur Diagnose von neurodegenerativen Erkrankungen**

(71) Anmelder: B.R.A.H.M.S. Aktiengesellschaft, 16761 Hennigsdorf (DE)
(72) Erfinder: Bergmann, Andreas, Dr., 12351 Berlin (DE); Struck, Joachim, Dr., 13465 Berlin (DE); Ernst, Andrea, 16761 Henningsdorf (DE)
(74) Vertreter: Andrae, Steffen

(57) **Zusammenfassung**

*In vitro* Verfahren zur Erkennung, zur Bestimmung des Schweregrads und zur Verlaufsbeurteilung und Prognose von neurodegenerativen Erkrankungen, bei dem man in einer biologischen Flüssigkeit eines Patienten, der an einer neurodegenerativen Erkrankung leidet oder bei dem Verdacht auf eine solche Erkrankung besteht, die Anwesenheit und/oder Konzentration der Carbamoylphosphat Synthetase 1 (CPS 1) bestimmt und auf der Basis der festgestellten Anwesenheit und/oder Konzentration von CPS 1 oder der Nichtnachweisbarkeit einer CPS 1-Immunreaktivität Schlüsse hinsichtlich des Vorliegens, des Verlaufs, des Schweregrads oder des Erfolgs einer Therapie der neurodegenerativen Erkrankung zieht.

## Beschreibung

Die vorliegende Erfindung betrifft ein neues in *vitro* Verfahren für die Diagnose von neurodegenerativen Erkrankungen, insbesondere von Demenzerkrankungen wie der Alzheimer Krankheit. Die Erfindung beruht darauf, dass überraschend festgestellt wurde, dass ein Enzym aus der Leber, nämlich das Enzym Carbamoylphosphat Synthetase 1 (E.C. 6.3.4.16, nachfolgend stets abgekürzt CPS 1), und/oder physiologisch auftretende CPS 1-Fragmente mit CPS 1-Immunreaktivität, in der Zirkulation von Alzheimer Patienten, insbesondere in Plasma oder Serum, signifikant erhöht gefunden wird und sich daher zur Verwendung als humoraler Biomarker in der medizinischen Diagnostik zum Nachweis von neurodegenerativen Erkrankungen eignet.

Im Rahmen der vorliegenden Erfindung werden dabei Begriffe wie "Diagnostik" oder "diagnostisch" als Oberbegriffe für medizinische Bestimmungen gebraucht, denen je nach dem klinischen Zustand des Patienten, bei dem die Bestimmung durchgeführt wird, und je nach den über ihn bereits verfügbaren Informationen, unterschiedliche Fragestellungen zugrunde liegen können und die insbesondere der Erkennung und Früherkennung, der Bestimmung des Schweregrads und der Verlaufsbeurteilung, auch der therapiebegleitenden Verlaufsbeurteilung, und der Prognose des zukünftigen Verlaufs einer Erkrankung dienen.

Dabei ist im vorliegenden Zusammenhang von besonderer Bedeutung, dass eine Diagnose auch eine Negativdiagnose sein kann, bei der man aufgrund der Nichtfeststellbarkeit eines bestimmten krankheitstypischen Merkmals, z.B. der Nichtnachweisbarkeit eines mit der betreffenden Krankheit assoziierten Biomarkers in einer Blutprobe eines Patienten, das Vorliegen einer bestimmten Erkrankung zuverlässig ausschließt.

Für die Negativdiagnose sind auch Biomarker von großem Wert, die bei mehreren verschiedenen Krankheiten erhöht gefunden werden können und daher allein, für sich genommen, noch keine positive Diagnose einer spezielle Krankheit ermöglichen - obwohl sie in der Regel bei Hinzuziehung weiterer klinischer oder biochemischer Parameter auch für die Positivdiagnose entscheidend sein können.

Die Erkrankungen, um deren Diagnose es bei der vorliegenden Erfindung geht, sind sich eher langsam entwickelnde, chronische neurodegenerative Erkrankungen von nichtinfektiöser Ätiologie, insbesondere präsenile Demenz-Erkrankungen.

Als Demenz-Erkrankungen (Dementia) werden generell Krankheiten bezeichnet, für die ein gemeinsames Merkmal der Verlust erworbener intellektueller Fähigkeiten, v.a. des Gedächtnisses, und des normalen Persönlichkeitsniveaus als Folge von Hirnschädigungen ist. Demenzerkrankungen sind in der Regel sich relativ langsam entwickelnde Krankheiten von chronischem Charakter. Treten Demenzerscheinungen vor dem hohen Alter im mittleren Lebensalter auf, werden sie als präsenile Demenz-Erkrankungen bezeichnet, und bei diesen unterscheidet man auf der Basis der für sie typischen Symptome und hirnpathologischen Veränderungen insbesondere die folgenden Erkrankungen bzw. Gruppen von Erkrankungen:

Die Alzheimer Demenz (AD) (Alzheimer Krankheit; Morbus Alzheimer) ist die häufigste neurodegenerative Demenz-Erkrankung und macht 2/3 aller Demenzfälle aus. AD zeichnet sich durch drei wichtige, allerdings erst post mortem mit Sicherheit feststellbare pathologische Merkmale aus: die Bildung von Amyloid-Plaques und neurofibrillären Bündeln sowie den Verlust an Nervenzellen (Übersicht s. SELKOE D. J. (2001). Alzheimer's disease: genes, proteins, and therapy. Physiological Reviews 81: 741-766). Amyloid-Plaques bestehen aus extraneuronalen Aggregaten des Amyloid-β-Proteins, während die Neurofibrillenbündel hauptsächlich Tau-Protein und Neurofilamente enthalten. Es wird vermutet, dass die Plaque- und Neurofibrillenbildung die Ursache für das Absterben von Nervenzellen ist.

Die wichtigsten Symptome von AD sind zunehmende Merkfähigkeits- und Denkstörungen bei relativ lang anhaltender Gemütsansprechbarkeit, wobei diese Symptome von weiteren weniger spezifischen Störungen begleitet werden, die die Abgrenzung der AD von anderen Demenzformen erschweren.

Die Demenz mit Lewy-Körperchen (dementia with lewy-bodies: DLB) ist nach der Alzheimer Demenz die zweithäufigste Ursache für eine demenzielle Erkrankung. Neuropathologisch ist die DLB durch das Auftreten von sogenannten Lewy-Körperchen im Hirnstamm und im Cortex charakterisiert. Diese LewyKörperchen bestehen überwiegend aus Aggregaten des präsynaptischen Proteins (α-Synuclein) und Ubiquitin. Die LewyBody-Pathologie kann in unterschiedlichem Ausmaß mit Alzheimer und Parkinson-typischen neuropathologischen Veränderungen assoziiert sein. So kommt es auch bei der DLB zur Bildung von beta-Amyloid und senilen Plaques, jedoch nicht zu Neurofibrillenbündeln (Übersicht s. MCKEITH I. G. (2002). Dementia with lewy bodies. British Journal of Psychiatry 180: 144-147; vgl. auch GELDMACHER D. S. (2004). Dementia with Lewy bodies: diagnosis and clinical approach. Cleveland clinic Journal of Medicine 71:789-800). Lewy-Körperchen sind auch im Gehirn von Patienten mit Morbus Parkinson vorhanden, wenn auch in einer unterschiedlichen Verteilung.

Kernsymptome von DLB sind eine progrediente kognitive Störung, Verwirrtheitsepisoden mit fluktuierender Aufmerksamkeits- und Bewusstseinslage, Parkinsonismus, häufige Stürze und Synkopen (anfallsartige, kurz dauernde Bewusstlosigkeit). Die Sensitivität und Spezifität der diagnostischen Kriterien zeigen durchgehend eine hohe Spezifität, aber zum Teil eine sehr niedrige Sensitivität. Das bedeutet, dass die DLB im klinischen Alltag häufig nicht diagnostiziert wird.

Die Frontotemporale Demenz (FTD) wird auch als Pick'sche Krankheit bezeichnet und macht ca. 20% der präsenilen Demenzerkrankungen aus. FTD ist teilweise genetisch bedingt und zählt zu den sogenannten Tauopathien, die sich durch eine Über- oder Unterexpression eines Tauprotein-Subtyps bzw. durch die Expression eines mutierten Tauproteins auszeichnen. Neuropathologisch kommt es zu einer lokalen Atrophie des frontalen und/oder temporalen Cortex sowie der Substantia Nigra und der Basalganglien. Dies hat unterschiedlich ausgeprägte Sprachstörungen, eine Wesensänderung sowie Verhaltensauffälligkeiten zur Folge. Insgesamt ist die FTD mit einer Sensitivität von 93% bei einer Spezifität von nur 23% unterdiagnostiziert, wobei die AD die häufigste Fehldiagnose darstellt.

Unter dem Begriff vaskuläre Demenz (vascular dementia; VAD) werden Erkrankungen zusammengefasst, bei denen eine Demenz aufgrund von Durchblutungsstörungen im Gehirn ausgelöst wird. Es gibt unterschiedliche Typen der VAD, von denen die Multi-Infarkt-Demenz (MID) und die subcorticale VAD (auch als Binswanger'sche Erkrankung bezeichnet) die häufigsten Formen darstellen.

Bei der Binswanger'schen Erkrankung handelt es sich um eine langsam progrediente demenzielle Entwicklung, die pathologisch durch cerebrovasculäre Läsionen in der weißen Hirnsubstanz charakterisiert ist. Klinisch resultiert dies in Verhaltensauffälligkeiten wie Agitation, Reizbarkeit, Depression und Euphorie sowie einer leichten Gedächtnisstörung.

Die Multi-Infarkt-Demenz entsteht allmählich als Folge von mehreren kleinen Schlaganfällen, auch als transiente ischämische Attacken (TIA) bezeichnet, die zum Untergang von Hirngewebe im Cortex und/oder subcortikalen Arealen führten. Die Schlaganfälle können auch gänzlich unbemerkt geblieben sein, die Demenz ist in diesem Falle die erste spürbare Folge. Bei Vorliegen einer MID kommt es zur stufenweisen Abnahme kognitiver Fähigkeiten, verbunden mit schweren Depressionen, Stimmungsschwankungen und Epilepsie.

Eine Diagnose auf Demenz-Erkrankungen erfolgt heutzutage überwiegend auf der Basis neuropsychologischer Untersuchungen und der Beobachtung der Krankheitsentwicklung und ihres Verlaufs, unter Heranziehung von Ausschlusskriterien für bestimmte Demenzformen. Diese Untersuchungen liefern in sehr vielen Fällen mehrdeutige Ergebnisse, die die o.g. Zahlen für die unterdiagnostizierten Demenzformen oder unrichtig diagnostizierten Fälle erklären. Die krankheitstypischen Gehirnveränderungen können an lebenden Patienten naturgemäß nicht direkt festgestellt werden, und apparatemedizinische Untersuchungen der Gehirnfunktionen mittels z.B. Röntgen- oder Kernspin-Tomographie sind aufwändig und teuer.

Für die Alzheimer-Diagnostik veröffentlichten das Ronald und Nancy Reagan Institut der Alzheimer Association und die NIA-Working Group Richtlinien für die Kriterien, die an einen idealen Biomarker zur Detektion der AD gestellt werden (7). Folgende Kriterien sollen im Idealfall durch den Biomarker erfüllt werden:
1. Er sollte hirnspezifisch sein und ein fundamentales Merkmal der Neuropathologie dieser Erkrankungen detektieren.
2. Die diagnostische Sensitivität und die Spezifität von mindestens 80% sollte gegeben sein.
3. Die krankheitsspezifische Veränderung des Biomarkers sollte sich in einem möglichst frühen Stadium der Erkrankung manifestieren, um mit geeigneten Therapiemaßnahmen beginnen zu können (GROWDON J. H., SELKOE D. J., ROSES A., TROJANOWSKI J. Q., DAVIES P., APPEL S. et al. [Working Group Advisory Committee]. (1998). Consensus report of the Working Group on Biological Markers of Alzheimer's Disease. [Ronald und Nancy Reagan Institute of the Alzheimer's Association and National Institute on Aging Working Group on Biological Biomarkers of Alzheimer's Disease]. Neurobiology of Aging 19: 109-116).

Bis jetzt gibt es jedoch keinen Biomarker, der im klinischen Alltag im Blut oder der cerebrospinalen Flüssigkeit mit ausreichender Sicherheit zur Früh- und Differentialdiagnose von AD herangezogen werden könnte und alle o.g. Kriterien erfüllt. Aktuell werden verschiedene potentielle Markerkandidaten untersucht, darunter Inflammationsmarker wie IL-6 und TNFα, Marker für oxidativen Stress wie 3-Nitrotyrosin, sowie Marker, die mit charakteristischen pathologischen Veränderungen der AD assoziiert sind, wie Amyloid β, das einen Hauptbestandteil der Amyloid-Plaques darstellt, und das Tau-Protein, das einen wesentlichen Bestandteil der Neurofibrillenbündel darstellt (vgl. die Übersichten in FRANK R. A., GALASKO D., HAMPEL H., HARDY J., DE LEON M. J., MEHTA P. D., ROGERS J., SIEMERS E., TROJANOWSKI J. Q. (2003). Biological markers for therapeutic trials in Alzheimer's disease. Proceedings of the biological markers working group; NIA initiative on neuroimaging in Alzheimer's disease. Neurobiology of Aging 24: 521-536; oder in TEUNIS-SEN C. E., DE VENTE J., STEINBUSCH H. W. M., DE BRUIJN C. (2002). Biochemical markers related to Alzheimer's dementia in serum and cerebrospinal fluid. Neurobiology of Aging 23:485-508).

Aus der WO 2003/089934 ist es ferner bekannt, dass in Serum und Plasma von Alzheimer Patienten das Peptid LASP-1, das auch in der Zirkulation von Sepsispatienten gefunden wird, signifikant erhöht ist. Die klinische Wertigkeit dieses Befundes ist gegenwärtig Gegenstand weiterführender Untersuchungen.

Es besteht derzeit weiterhin ein aktueller Bedarf nach ergänzenden, valide Laborbefunde liefernden Untersuchungsmethoden, die auf einer Bestimmung von als Biomarker für Demenzerkrankungen, insbesondere für die Alzheimer Demenz (AD), geeigneten Substanzen in Blut- bzw. Plasmaproben beruhen und bei Patienten, bei denen der Verdacht auf Vorliegen einer Demenzerkrankung, insbesondere von AD, besteht, für eine Positivdiagnose und/oder für eine negative Ausschlussdiagnose geeignet sind.

Die vorliegende Erfindung stellt eine solche Untersuchungsmethode in Form eines *in vitro* Verfahrens zur Erkennung, zur Bestimmung des Schweregrads und zur Verlaufsbeurteilung und Prognose von neurodegenerativen Erkrankungen bereit, das dadurch gekennzeichnet ist, dass man in einer biologischen Flüssigkeit eines Patienten, der an einer neurodegenerativen Erkrankung leidet oder bei dem Verdacht auf eine solche Erkrankung besteht, die Anwesenheit und/oder Konzentration des Enzyms Carbamoylphosphat Synthetase 1 (CPS 1), und/oder physiologisch auftretender CPS 1-Fragmente mit CPS 1-Immunreaktivität, bestimmt und auf der Basis der festgestellten Anwesenheit und/oder Konzentration von CPS 1 oder der Nichtnachweisbarkeit einer CPS 1-Immunreaktivität Schlüsse hinsichtlich des Vorliegens, des Verlaufs, des Schweregrads oder des Erfolgs einer Therapie der neurodegenerativen Erkrankung zieht.

Vorteilhafte bzw. bevorzugte Ausgestaltungen eines Verfahrens gemäß Anspruch 1 sind in den Unteransprüchen 2 bis 12 wiedergegeben.

Der vorliegenden Erfindung liegen völlig überraschende Befunde einer systematischen Untersuchung einer großen Zahl von Seren und Plasmen von Patienten mit der Diagnose "wahrscheinlich Alzheimer Demenz" mit Hilfe von neuartigen Immunoassays zur Bestimmung verschiedener Biomarker zugrunde, die sich bei der Anmelderin in der Entwicklung und klinischen Erprobung befinden und z.T. Gegenstand veröffentlichter oder noch unveröffentlichter früherer Patentanmeldungen der Anmelderin sind.

Dabei ergaben die nachfolgend im experimentellen Teil beschriebenen Messergebnisse in EDTA-Plasmaproben von augenscheinlich gesunden Normalpersonen und Alzheimer-Patienten eine klare, diagnostisch signifikante Korrelation zwischen den für CPS-1 gefundenen Konzentrationen und dem Vorliegen von Demenzsymptomen, die zur Diagnose "wahrscheinlich Alzheimer" geführt hatten.

Obwohl die Untersuchungen bisher auf Plasmaproben von Alzheimer Patienten beschränkt waren, gehen die Erfinder davon aus, dass - möglicherweise mit unterschiedlichen typischen Konzentrationsbereichen - auch bei anderen neuroinflammatorischen Demenzformen, insbesondere bei vaskulärer Demenz (VAD) und Demenz mit Lewy-Körperchen (DLB), charakteristische Erhöhungen CPS 1 Konzentrationen in Patientenplasmen feststellbar sein müssten.

Das für die im experimentellen Teil beschriebenen Messungen eingesetzte Bestimmungsverfahren für CPS-1 in Patientenplasmen ist eine Abwandlung eines nichtkompetitiven immunoluminometrischen Sandwichassays zur Bestimmung von CPS-1, wie er in näheren Einzelheiten in der WO 03/089933 bzw. der EP 1 497 662 A1 der Anmelderin beschrieben wird.

Für die medizinische Diagnostik spielten CPS 1 bzw. CPS 1-Fragmente mit CPS 1-Immunreaktivität traditionell keine praktische Rolle. Auf dem Gebiet der Diagnostik von neurodegenerativen Erkrankungen wurde das Leberenzym CPS 1 noch nie als möglicher humoraler Biomarker in Betracht gezogen.

Das Enzym CPS 1 (E.C. 6.3.4.16) selbst ist jedoch seit langem gut bekannt. Es katalysiert die Umwandlung von Ammoniak, Bicarbonat und 2 ATP unter Bildung von Carbamoylphosphat im ersten Schritt des Harnstoffzyklus. Es spielt dabei auch eine Rolle bei der Biosynthese von Arginin, das seinerseits ein Substrat für die Biosynthese von NO, z.B. bei einem Endotoxin-Schock, ist (vgl. Shoko Tabuchi et al., Regulation of Genes for Inducible Nitric Oxide Synthase and Urea Cycle Enzymes in Rat Liver in Endotoxin Shock, Biochemical and Biophysical Research Communications 268, 221-224 (2000)). CPS 1 ist von dem cytosolischen Enzym CPS 2 (E.C. 2.7.2.5.) zu unterscheiden, das ebenfalls eine Rolle im Harnstoffzyklus spielt, jedoch das Substrat Glutamin verarbeitet. Es ist bekannt, dass CPS 1 in Mitochondrien lokalisiert ist und im Lebergewebe in dieser Form in großen Mengen (es macht von 2-6% des Leber-Gesamtproteins aus) vorkommt. Seine Aminosäuresequenz und genetische Lokalisierung ist seit langem bekannt (vgl. Haraguchi Y. et al, Cloning and sequence of a cDNA encoding human carbamyl phosphate synthetase I: molecular analysis of hyperammonemia, Gene 1991, Nov. 1; 107 (2):335-340; vgl. auch die Veröffentlichung WO 03/089933 A1 der Anmelderin). Zu seiner physiologischen Rolle kann verwiesen werden auf Reviewartikel wie z.B. H.M.Holder et al., Carbamoyl phosphate synthetase: an amazing biochemical odyssey from substrate to product, CMLS, Cell.Mol.Life Sci. 56 (1999) 507-522 und die darin referierte Literatur, sowie die Einleitung der Veröffentlichung Mikiko Ozaki et al., Enzyme-Linked Immunosorbent Assay of Carbamoylphosphate Synthetase I: Plasma Enzyme in Rat Experimental Hepatitis and Its Clearance, Enzyme Protein 1994, 95:48:213-221.

Die in der vorliegenden Anmeldung beschriebenen Messungen stellen den ersten Bericht über ein Auftreten von CPS 1 in der Zirkulation von Patienten mit neurodegenerativen Erkrankungen, insbesondere Patienten mit der Diagnose Alzheimer Demenz, dar. Bisher wurde CPS 1, und auch nur in Untersuchungen der Anmelderin, lediglich in Serum bzw. Plasma von Sepsispatienten bestimmt (vgl. WO 03/089933 A1). Sepsispatienten, deren hochakute potentiell lebensbedrohende Erkrankung typischerweise in Intensivstationen überwacht und behandelt wird, stellen eine von Patienten mit neurodegenerativen Erkrankungen, die an einer sich über lange Zeiträume entwickelnden Erkrankung leiden, klar unterschiedene Patientenpopulation dar.

Bei der Bestimmung von CPS 1 bzw. CPS 1-Immunreaktivität als humoraler Biomarker in Patientenseren gemäß der vorliegenden Erfindung kann grundsätzlich so vorgegangen werden, wie das in der Veröffentlichung WO 03/089933 A1 der Anmelderin im Zusammenhang mit der Bestimmung von CPS 1 als Sepsismarker beschrieben wurde. Das im experimentellen Teil der vorliegenden Anmeldung beschriebene Bestimmmungsverfahren, das zur Prüfung von Seren bzw. Plasmen von Patienten mit der Diagnose "wahrschenlich Alzheimer Demenz" auf die Anwesenheit von CPS 1 bzw. CPS 1-Immunreaktivität eingesetzt wurde, ist eine Abwandlung des Verfahrens, das in der o.g. Anmeldung WO 03/089933 A1 der Anmelderin beschrieben wurde. Die Abwandlung berücksichtigt die in der WO 03/089933 A1 offenbarte Tatsache, dass die in der Zirkulation zu findende Spezies mit CPS 1-Immunreaktivität wenigstens überwiegend das vollständige, oder wenigstens weitgehend vollständige, Enzym CPS 1 selbst ist. Neben einem an die Aminosäuren 184-199 der Aminosäuresequenz der humanen CPS 1 bindenden ersten Antikörper, wie er auch in der o.g. WO 03/089933 verwendet wird, wird ein zweiter Antikörper verwendet, der an die Aminosäuren 781 bis 794 der genannten Sequenz bindet.

Als "Verwendung von CPS 1 als Biomarker" im Sinne der vorliegenden Anmeldung ist nicht nur die direkte immunologische Bestimmung von CPS 1 in in *vitro* Proben für diagnostische Zwecke anzusehen, sondern auch eine Verwendung von CPS 1 bzw. CPS 1-Fragmenten, oder von Antikörpern zu deren selektiver Bestimmung, zur Herstellung von Assaykits, oder eine Verwendung zur Erzeugung von Assaykomponenten, z.B. von poly- oder monoklonalen Antikörpern, die z.B. in immobilisierter und/oder markierter Form in der Regel ebenfalls in Assaykits für die angegebenen Erkrankungen vorgesehen sind, oder von Standard- und Bezugssubstanzen.

Es ist zusätzlich ausdrücklich darauf hinzuweisen, dass bei der erfindungsgemäßen Bestimmung von CPS 1 oder CPS 1-Immunreaktivität je nach Assaydesign eine gleichzeitige Bestimmung sowohl von CPS 1 in Form des im wesentlichen vollständigen Moleküls als auch in Form von in der biologischen Flüssigkeit möglicherweise vorhandenen anderen, kürzeren Bruchstücken (physiologisch vorkommenden Teilpeptiden) der vollständigen CPS 1 erfolgen kann. Wenn in der vorliegenden Anmeldung von einer Bestimmung von "CPS 1-Immunreaktivität" gesprochen wird, soll das diesem messtechnischen Umstand Rechnung tragen, damit eine unsachgemäße einengende Auslegung der Lehre der vorliegenden Erfindung vermieden wird.

An Stelle der Bestimmung von CPS 1 oder CPS 1-Immunreaktivität soll für diagnostische Zwecke die CPS 1-Bestimmung ggf. auch indirekt als Bestimmung einer Enzymaktivität, die der CPS 1-Aktivität oder der Restaktivität der CPS 1-Fragmente im Blut entspricht, erfolgen können. Da CPS 1 bei Gesunden in der Zirkulation nicht auftritt, kann eine messbare CPS 1 Enzymaktivität im Blut eines Patienten ein diagnostisch signifikantes Indiz für eine ernsthafte Störung der gesundheitlichen Unversehrtheit des Patienten sein. Es sei an dieser Stelle auch noch darauf hingewiesen, dass die Aktivität eines Enzyms, das normalerweise im Zellinneren lokalisiert ist und nur dort seine funktionsgerechte Wirkung entfaltet, in der Zirkulation per se negativ zu bewerten ist und daher auch als solche zu einer Verschärfung eines Krankheitszustands beitragen kann.

Die eigentliche CPS 1-Bestimmung kann nicht nur auf die im experimentellen Teil konkret beschriebene Weise erfolgen, sondern auf irgendeine geeignete, an sich bekannte Weise, wobei Immunoassays eines geeigneten Assaydesigns bevorzugt sind.

Die Verfahren zur Bestimmung von CPS 1-Immunreaktivität in einer biologischen Probe können beliebige bekannte Verfahren der Immundiagnostik sein, die zum Nachweis und zur Messung von Antigenen angewandt werden. Vorzugsweise wird CPS 1 mit Hilfe eines Ligandenbindungsassays bestimmt, bei dem man zur Bindung und Markierung geeignete spezifische Antikörper in immobilisierter Form bzw. markierter bzw. markierbarer Form verwendet.

Auch kompetitive Assayformate können besondere Vorteile bieten. Vorzugsweise wird dabei nicht mit einer Enyzmmarkierung gearbeitet, sondern es wird eine andere Markierung gewählt, z.B. eine Markierung für eine Chemilumineszenz-Nachweisreaktion, z.B. ein Akridiniumester. Selbstverständlich ist vorzugsweise ein solcher Assay zur CPS 1-Bestimmung zu verwenden, der die benötigte hohe Sensitivität im Bereich der bei neurodegenerativen Erkrankungen vorkommenden CPS 1-Konzentrationen gewährleistet und eine Separierung der Messignale vom Assay-Hintergrund ermöglicht.

Das Bestimmungsverfahren kann an die Chip-Technologie angepaßt sein oder als Schnelltest (Point-of-Care-Test) ausgestaltet werden.

Bei einer bevorzugten Ausführungsform wird die immundiagnostische Bestimmung als heterogener Sandwich-Immunoassay durchgeführt, bei dem einer der Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während der andere Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Die beiden Antikörper können auch im Falle heterogener Sandwich-Immunoassays Teile eines Nachweissystems der nachfolgend im Zusammenhang mit homogenen Assays beschriebenen Art aufweisen.

Das erfindungsgemäße Verfahren kann ferner als homogenes Verfahren ausgestaltet werden, bei dem die aus den beiden Antikörpern und dem nachzuweisenden CPS 1 gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TRACE^{®} (Time Resolved Amplified Cryptate Emission) bzw. KRYPTOR^{®} angebotene Technologie zu verweisen, die die Lehren der o.g. Anmeldungen umsetzt.

Der Inhalt der genannten älteren Anmeldung (WO 03/089933 A1) der Anmelderin ist durch die ausdrückliche Bezugnahme auf diese Anmeldungen als Teil der Offenbarung der vorliegenden Anmeldung anzusehen.

Nachfolgend wird die Erfindung anhand von Messergebnissen und von zwei Figuren noch näher erläutert.

Es zeigen:
- Figur 1: eine typische Eichkurve für die Bestimmung von CPS-1 in Patientenplasmen mit dem im experimentellen Teil näher beschriebenen Assay;
- Figur 2: die Ergebnisse der Messung der CPS-1 Konzentrationen in EDTA-Plasmen von Patienten mit der Diagnose "wahrscheinlich Alzheimer Demenz" (wsAD), gegenübergestellt den Ergebnissen gleicher Messungen bei altersgematchten Kontrollpersonen ohne Zeichen von Alzheimer Demenz.

### ExperimentellerTeil

### Assaybeschreibung

### Herstellung der Antikörper

### a) Immunogene

Es wurden 2 verschiedene Peptidsequenzen aus der vollständigen Sequenz der humanen CPS 1 ausgewählt, und zwar eine erste Peptidsequenz 1 (EFEGQPVDFVDPNKQN), die den Aminosäuren 184-199 der Sequenz der humanen CPS 1 entspricht (vgl. Peptid PCEN17 gemäß WO 03/089933), und eine zweite Peptidsequenz (FHGTSSRIGSSMKS), die den Aminosäuren 781-794 der Sequenz des humanen CPS 1 entspricht. Jedes Peptid wurde in einer mit einem aminoterminalen Cysteinrest (Cys0) versehenen Form von der Firma Jerini (Berlin, Deutschland) synthetisiert. Die synthetisierten, für die nachfolgenden Immunisierungen verwendeten Peptide sind im Sequenzprotokoll als SEQ ID NO:1 bzw. SEQ ID NO:2 gezeigt.

### b) Antikörper

Für die Immunisierung wurden die beiden synthetisierten Peptide mit dem Hämocyanin aus *Limulus polyphemus* konjugiert, und es wurden, wie auch in der WO 03/089933 beschrieben, polyklonale Antikörper in Schafen durch die Firma Ltd. Micropharm (Carmarthenshire, Großbritannien) produziert.

### 2. Reinigung der Antikörper

Die Antikörper wurden mittels ligandenspezifischer Affinitätsreinigung purifiziert. Hierfür wurden die Cys (0)-Peptide 1 und 2 zunächst an SulfoLink-Gel der Firma Pierce (Boston, USA) gekoppelt. Die Bindung erfolgte nach der Vorschrift des Herstellers.

Ds Vorgehen war wie folgt: Polycarbonat-Säulen (15mm x 80mm) wurden mit 5 ml Affinitätsmatrix befüllt. Nach Äquilibrierung der Säulen mit PBS (136 mM NaCl, 1,5 mM KH₂PO₄, 20,4 mM Na₂HPO₄, 2H₂O, 2,7 mM KCl, pH 7,2) wurden 5 mg der jeweiligen Peptide abgewogen, in PBS gelöst, und auf die verschlossenen Säulen gegeben. Das Gelmaterial wurde durch Schwenken homogenisiert. Nach 15minütiger Inkubation bei Raumtemperatur und Absetzen des Gelmaterials wurden die Säulen mit 5mal 3ml PBS gewaschen. Zur Absättigung freier Bindungsstellen wurden dem Säulenmaterial je 5 ml einer 50 mM L-Cysteinlösung zugesetzt, und das Gelmaterial wurde nach Homogenisierung erneut bei Raumtemperatur für 15 min inkubiert. Nach Absetzen des Gelmaterials wurde jede Säule 6mal mit 5ml einer 1M NaCl-Lösung gespült und anschließend nochmals mit PBS gespült.

Das Gelmaterial wurde mit 25ml des jeweiligen Antiserumpools gemischt und über Nacht bei Raumtemperatur unter leichtem Schwenken inkubiert. Die Serum-Gel-Gemische wurden in Polycarbonat-Säulen überführt, und überschüssiges Serum wurde entfernt. Die Säulen wurden anschließend mit 250 ml PBS gewaschen, um nicht gebundene Serumproteine zu entfernen. Die Desorption der gebundenen Antikörper erfolgte durch Elution der Säule mit 50 mM Citronensäure (pH 2,2). Das Eluat wurde in Fraktionen ä 1 ml aufgefangen. Die Proteinkonzentration jeder Fraktion wurde mit Hilfe des BCA-Proteinassay-Kits der Firma Perbio (Bonn, Deutschland) bestimmt, und die Fraktionen mit einem Proteingehalt > lmg/ml wurden vereinigt. Die affinitätsgereinigten Antikörper wurden mittels Dialyse in PBS umgepuffert und nach erneuter Bestimmung des Proteingehalts anschließend bei 4°C gelagert.

### 3. Immobilisierung / Markierung der Antikörper

Die aufgereinigten Antikörper gegen das Peptid, das der Aminosäuresequenz 781-794 entspricht, wurden auf Polystyrolröhrchen (Startubes, 12 mm x 75 mm, Firma Greiner, Deutschland) immobilisiert. Hierfür wurden die Antikörperlösungen auf eine Proteinkonzentration von 6,7 µg/ml mit PBS verdünnt und 300 µl pro Röhrchen pipettiert (entspricht 2 µg Antikörper pro Röhrchen). Diese wurden für 20 h bei Raumtemperatur inkubiert und anschließend 3mal mit je 4 ml PBS gewaschen. Bis zur weiteren Verwendung wurden die Röhrchen bei 4°C gelagert.

Der Antikörper gegen das Peptid, das der Aminosäuresequenz 184-199 entspricht, (1 mg/ml in PBS) wurde mit Acridiniumester-N-Hydroxy-Succinimid (1 mg/ml in Acetonitril, Firma InVent, Hennigsdorf Deutschland) lumineszenzmarkiert. Für die Markierung wurden 200 µl Antikörper mit 4 µl Acridiniumester gemischt, für 20 min inkubiert, und freie Acridiniumesterbindungen wurden durch Zugabe von 40µl einer 50mM Glycin-Lösung abgesättigt. Der Markierungsansatz wurde mittels HPLC an einer BioSil 400-Gelfiltrations-Säule (Firma BioRad, München, Deutschland) von freiem Acridiniumester getrennt. Als Laufmittel wurde PBS verwendet.

### 4. Relative Kalibrierung

Um die relativen CPS 1-Konzentrationen bestimmen zu können, wurde als Standardmaterial ein Pool von Plasmen von humanen Patienten mit SIRS mit besonders hohen CPS 1-Konzentrationen verwendet. Die CPS 1-Konzentration dieses Pools wurde arbiträr auf 150 U/ml festgesetzt. Ausgehend von diesem Pool wurden durch serielle Verdünnung mit CPS 1-freiem humanem Plasma von Gesunden Standards hergestellt, denen arbiträre Konzentrationen entsprechend ihrer Verdünnung zugeschrieben wurden.

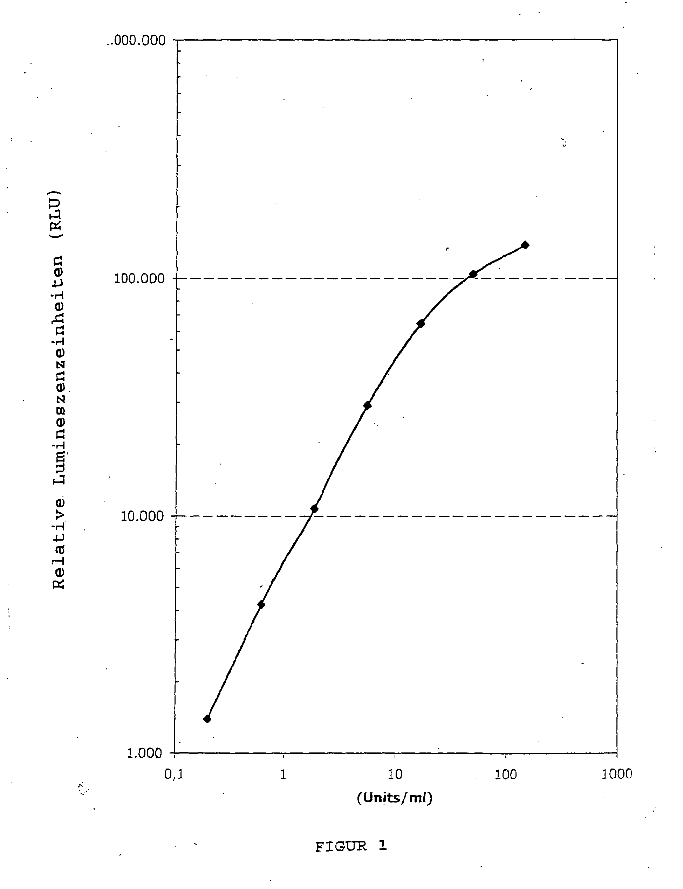

Eine typische Standardkurve mit den entsprechenden relativen Konzentrationen ist in Figur 1 dargestellt. Die analytische Assay-Sensitivität liegt bei 0,9 U/ml.

### 5. Assay-Durchführung

50µl einer Plasma-Probe und 100µl PBS-Puffer (mit 10 mM EDTA) wurden pro Antikörper-beschichtetes Röhrchen pipettiert und 16 h bei Raumtemperatur inkubiert. Nach 3maligem Waschen mit je 1 ml PBS wurden pro Röhrchen 15 ng des markierten Antikörpers (in 200µl PBS-Puffer, 10mM EDTA) zugesetzt. Die Röhrchen wurden für weitere 2h inkubiert, und anschließend wurde ungebundener Tracer-Antikörper durch 5maliges Waschen mit je 1ml PBS entfernt. Am Röhrchen gebundener markierter Antikörper wurde mittels Lumineszenzmessung in einem Luminometer (Berthold LB 952T/16) quantifiziert.

### Messung von CPS 1 im Plasma von gesunden Kontrollen und Patienten mit einer wahrscheinlichen Alzheimer-Demenz

Mit dem oben beschriebenen Assay wurden auf die angegebene Weise relative CPS 1-Konzentrationen in 115 EDTA-Plasma-Proben von augenscheinlich gesunden Kontrollpersonen und in 105 EDTA-Plasma-Proben von Patienten mit einer wahrscheinlichen Alzheimer Demenz (wsAD-Patienten; klinisch diagnostiziert anhand der NINCDS/ARDA-Kriterien, s. McKhann G., Drachman G., Folstein M., Katzman R., Price D., Stadlan E.M.: Clinical diagnosis of Alzheimer's disease: Report of the NINCDS/ARDA workgroup under the auspices of the Departement of health and human services task force on Alzheimer's disease, Neurology 34:939-944 (1984)) bestimmt.

Die Kontrollen weisen einen Altersmedian von 67,9 ± 12,4 Jahren auf, und die wsAD-Patienten einen Altersmedian von 73,3 ± 9,4 Jahren auf.

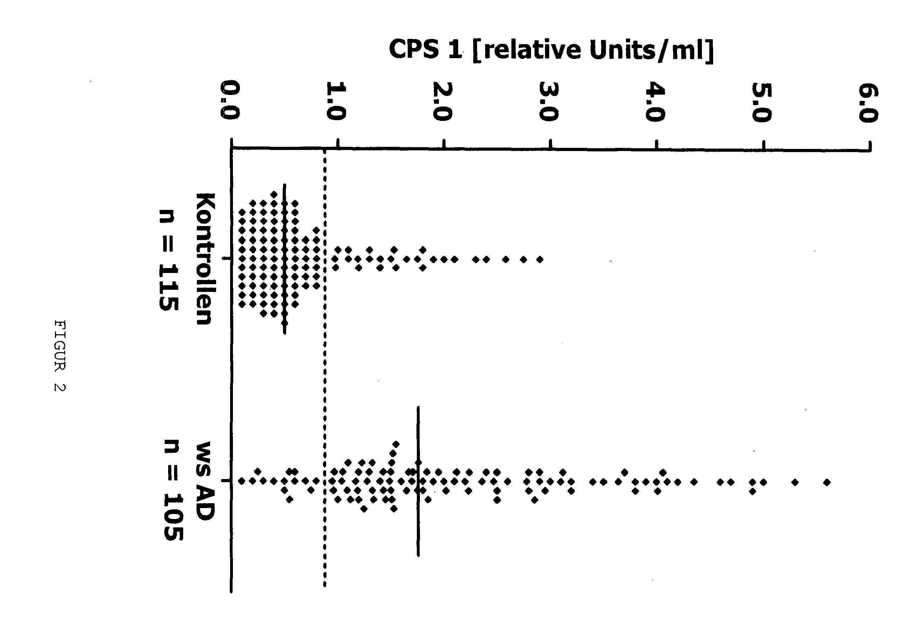

Der Cut-off des Assays wurde auf 0,9 U/ml (relative Einheiten pro ml) festgelegt, was der analytischen Sensitivität des Assays entspricht. Bei 91 von 105 wsAD-Patienten wurden relative CPS 1-Konzentrationen von mehr als 0,9 U/ml gemessen. Dagegen liegen die messbaren CPS 1-Konzentrationen von 89 von 115 Kontrollen unter 0,9 U/ml. Die Ergebnisse sind graphisch in Figur 2 gezeigt.

Damit können Alzheimer-Patienten von Kontrollen mit einer Spezifität von 77,4% und einer Sensitivität von 86,7% unterschieden werden. Der Unterschied zwischen beiden Gruppen ist hoch signifikant (P-Wert <0,0001). Die mediale Konzentration der Kontrollen lag unterhalb der Sensitivität des Tests, wohingegen Alzheimer-Patienten eine mediale CPS 1-Konzentration von 1,76 U/ml aufwiesen.

## Patentansprüche

1. *In* vitro Verfahren zur Erkennung, zur Bestimmung des Schweregrads und zur Verlaufsbeurteilung und Prognose von neurodegenerativen Erkrankungen, **dadurch gekennzeichnet, dass** man in einer biologischen Flüssigkeit eines Patienten, der an einer neurodegenerativen Erkrankung leidet oder bei dem Verdacht auf eine solche Erkrankung besteht, die Anwesenheit und/oder Konzentration der Carbamoylphosphat Synthetase 1 (CPS 1), und/oder von physiologisch auftretenden CPS 1-Fragmenten mit CPS 1-Immunreaktivität, bestimmt und auf der Basis der festgestellten Anwesenheit und/oder Konzentration von CPS 1 oder der Nichtnachweisbarkeit einer CPS 1-Immunreaktivität Schlüsse hinsichtlich des Vorliegens, des Verlaufs, des Schweregrads oder des Erfolgs einer Therapie der neurodegenerativen Erkrankung zieht.

2. Verfahren nach Anspruch 1, **dadurch gekennzeichnet, dass** das Bestimmungsverfahren ein immundiagnostisches Bestimmungsverfahren ist.

3. Verfahren nach einem der Ansprüche 1 oder 2, **dadurch gekennzeichnet, dass** das immundiagnostische Bestimmungsverfahren ein Immunoassay vom Sandwichtyp ist.

4. Verfahren nach Anspruch 3, **dadurch gekennzeichnet, dass** man im Plasma eines Patienten CPS 1 mit Hilfe eines Sandwichassays bestimmt, der CPS 1 sowie CPS 1-Fragmente, die mindestens die Aminosäuren 184 bis 794 der humanen CPS 1 aufweisen, erkennt.

5. Verfahren nach Anspruch 1, **dadurch gekennzeichnet, dass** die CPS 1-Bestimmung als Bestimmung der CPS 1-Enzymaktivität in Blut, Plasma oder Serum erfolgt.

6. Verfahren nach einem der Ansprüche 1 bis 5, **dadurch gekennzeichnet**, die neurodegenerative Erkrankung eine präsenile Demenzerkrankung ist, die ausgewählt ist aus einer Gruppe, die die Alzheimer Demenz (AD), Demenz mit Lewy-Körperchen (DLB), frontotemporale Demenz (FTD) und verschiedene Formen der vaskulären Demenz (VD) umfaßt.

7. Verfahren nach Anspruch 6, **dadurch gekennzeichnet, dass** es im Rahmen der Diagnostik der Alzheimer Demenz durchgeführt wird.

8. Verfahren nach einem der Ansprüche 1 bis 7, **dadurch gekennzeichnet, dass** es im Rahmen einer Multiparameter-Bestimmung durchgeführt wird, bei der gleichzeitig mindestens ein weiterer für das jeweilige Krankheitsbild aussagekräftiger biochemischer oder physiologischer Parameter bestimmt wird und bei der ein Messergebnis in Form eines Satzes von mindestens zwei Messgrößen gewonnen wird, der zur Feindiagnostik der neurodegenerativen Erkrankung ausgewertet wird.

9. Verfahren nach Anspruch 8, **dadurch gekennzeichnet, dass** im Rahmen der Multiparameter-Bestimmung neben der Bestimmung der CPS 1 wenigstens ein weiterer biochemischer Parameter bestimmt wird, der ausgewählt ist aus den Gruppen der Entzündungsmediatoren, Komplementkomponenten, Cytokine, Chemokine, der Blutkoagulanzien und fibrinolytischen Faktoren, Akutphasenproteine und radikalischen Verbindungen.

10. Verfahren nach Anspruch 8, **dadurch gekennzeichnet, dass** als mindestens ein weiterer biochemischer Parameter das Peptid LASP-1, ein physiologisch inaktives Präproadrenomedullin-Teilpeptid, Apolipoprotein A1, Apolipoprotein E4 und/oder Cu/Zn-Superoxid-Dismutase bestimmt wird.

11. Verfahren nach Anspruch 8, 9 oder 10, **dadurch gekennzeichnet, dass** die Multiparameter-Bestimmung als Simultanbestimmung,mittels einer Chiptechnologie-Messvorrichtung oder einer immunchromatographischen Messvorrichtung erfolgt.

12. Verfahren nach Anspruch einem der Ansprüche 8 bis 11, **dadurch gekennzeichnet, dass** die Auswertung des komplexen Messergebnisses der Multiparameter-Bestimmung mit Hilfe eines Computerprogramms erfolgt.


SEQUENCE LISTING

<110>  B.R.A.H.M.S Aktiengesellschaft

<120>  In vitro Verfahren zur Diagnose von neurodegenerativen
       Erkrankungen

<130>  4737EP2

<140>
<141>

<160>  2

<170>  PatentIn version 3.2

<210>  1
<211>  17
<212>  PRT
<213>  Artificial

<220>
<223>  Synthetic

<400>  1

Cys Glu Phe Glu Gly Gln Pro Val Asp Phe Val Asp Pro Asn Lys Gln
1               5                   10                  15

Asn

<210>  2
<211>  15
<212>  PRT
<213>  Artificial

<220>
<223>  Synthetic

<400>  2

Cys Phe His Gly Thr Ser Ser Arg Ile Gly Ser Ser Met Lys Ser
1               5                   10                  15